# EUROPEAN PATENT APPLICATION

(11) **EP 1 938 767 A1**
(43) Date of publication of application: **02.07.2008**
(21) Application number: 08004667.5
(22) Date of filing: 13.05.2004
(51) Int. Cl.: A61B 18/12

(54) **System for activating an electrosurgical instrument**

(30) Priority: 15.05.2003 US 470633 P
(62) Divisional of application: 04011375.5
(71) Applicant: Covidien AG, 8212 Neuhausen am Rheinfall (CH)
(72) Inventor: Blaha, Derek M., Longmont, CO 80503 (US)
(74) Representative: HOFFMANN EITLE

(57) **Abstract**

A system is disclosed for activating an electrosurgical instrument. The system includes an electrosurgical assembly, a control assembly, and an activation device. The electrosurgical assembly includes an electrosurgical instrument and a driver circuit operatively coupled to the electrosurgical instrument. A controller circuit, a safety circuit, and a latch circuit are included within the control assembly and interface between the electrosurgical assembly and an activation device. Instrument data and supervisory monitoring data originate in the electrosurgical assembly, and they are communicated to the controller circuit and the safety circuit. The electrical cooperation among the controller circuit, safety circuit, and latch circuit enables a user to selectively use the instrument in an "on/off" mode or in a "continuous" mode upon actuation of the same activation switch.

## Description

This application claims priority to an application entitled "SYSTEM FOR ACTIVIATING AN ELECTROSURGICAL INSTRUMENT" filed in the United States Patent and Trademark Office on May 15, 2003 and assigned Serial No. 60/470,633, the contents of which are hereby incorporated by reference.

### BACKGROUND OF THE INVENTION

### 1. Technical Field

The present disclosure relates to switching systems for activating electrosurgical instruments. More particularly, the present disclosure relates to a switching system for activating an electrosurgical instrument using a mechanical or electro-mechanical switch, which enables selective control over the electrosurgical instrument from an on/off mode to a continuous activation mode.

### 2. Background of Related Art

In conventional electrosurgical systems, electrosurgical instruments are routinely activated by operating personnel employing actuation mechanisms, or control assemblies, including activation devices such as hand switches, foot switches, pushbuttons, and the like. A number of electrosurgical instruments require continuous actuation of the actuation mechanism by the operator for the electrosurgical instruments' operation. During long surgical procedures, the operator may become fatigued or stressed causing the operator to interrupt the actuation of the actuation mechanism inadvertently. As a result, the electrosurgical instrument will stop operating during part of a surgical procedure.

Therefore, a need exists for a system to activate an electrosurgical instrument that overcomes one or more deficiencies of conventional electrosurgical systems. Furthermore, a need exists for a system for activating an electrosurgical instrument having an improved control assembly and a system for activating an electrosurgical instrument that reduces operator stress and/or fatigue.

### SUMMARY

An electrosurgical control system is hereinafter disclosed. The electrosurgical control system includes an electrosurgical assembly that is operatively coupled to a control assembly. An activation device is further included and is operatively coupled to the control assembly. The electrosurgical assembly includes an electrosurgical instrument operatively coupled to a driver circuit where the driver circuit provides a supply signal for operating the electrosurgical instrument. It is possible to include the driver circuit within the electrosurgical instrument or provide the driver circuit located remotely from the electrosurgical instrument. The driver circuit and the electrosurgical instrument provide feedback, instrument monitoring, and supervisory monitoring information to a controller circuit and a safety circuit within the control assembly. The control assembly further includes a switching circuit for controlling an activation operation, e.g., on/off or continuous, of the electrosurgical instrument.

The controller circuit communicates a power signal and a control signal to the electrosurgical assembly, and in particular to the driver circuit of the electrosurgical assembly through a connection. Typically, the connection is a cable including a plurality of wires, but may also be a radio frequency or infrared communication arrangement. In addition, the controller circuit receives an instrument signal from the electrosurgical instrument and a monitoring signal from the electrosurgical instrument and the driver circuit, thereby permitting the controller circuit to monitor conditions in the electrosurgical assembly and to adjust the power and control signals provided to the electrosurgical assembly accordingly. The controller circuit may include electro-mechanical, electrical, and/or electronic components for receiving the various inputs, processing the inputted data, and/or providing the power and control signal outputs.

The safety circuit provides a safety or control signal to the controller circuit. The instrument signal, the monitoring signal, and a latch signal are inputted into the safety circuit where they are processed prior to the safety circuit providing the safety signal output. Preferably, the inputs are processed by a logic circuit within the safety circuit where the logic circuit includes electro-mechanical, electrical, and/or electronic components. Included in the safety circuit is stored data relating to the operating parameters selected by the operator. This stored data is compared to the inputted signals and processed by the logic circuit. When the selected operational parameters are present, the safety circuit generates and provides the safety signal to the controller circuit, thereby permitting operation of the electrosurgical assembly. Additionally, the safety circuit may provide audible and/or visual indications to the operator as to the present status of the electrosurgical control system.

The switching circuit provides the latch signal to the respective input of the safety circuit and is responsive to an input trigger signal from the activation device. Operationally, the switching circuit may include electro-mechanical, electrical, and/or electronic components. A programmable latch circuit is further included in the switching circuit. The latch circuit has a variable time delay included. Upon actuation of the activation mechanism (e.g. a hand switch, a foot switch, or a button), the switching circuit is activated and provides the latch signal to the safety circuit, e.g., in an on/off mode. Upon activation of the activation mechanism for a predetermined time interval, e.g., a predetermined time delay" the latch circuit latches and provides a continuous latch signal to the safety circuit even if activation device no longer provides the trigger signal, e.g., when the activation device is not activated. In this manner, the electrosurgical instrument operates in a continuous operation mode without further activation.

According to an aspect of the present disclosure, an electrosurgical generator is provided including a control assembly for controlling an operation of an electrosurgical assembly, the electrosurgical assembly performing a surgical procedure, wherein upon the control assembly receiving a single trigger signal, the control assembly activates the electrosurgical assembly in a continuous operation mode.

According to a further embodiment, a method for controlling an electrosurgical instrument in a continuous operating mode is provided. The method comprises the steps of activating an activation device for a predetermined period of time; generating a latch signal after the predetermined period of time; generating a control signal upon receiving the latch signal to activate the electrosurgical instrument; and deactivating the activation device, wherein the electrosurgical instrument will operate in a continuous operating mode after the deactivation.

### BRIEF DESCRIPTION OF THE DRAWINGS

Preferred embodiments of the presently disclosed system for activating an electrosurgical instrument are described herein with reference to the drawing figure, wherein:

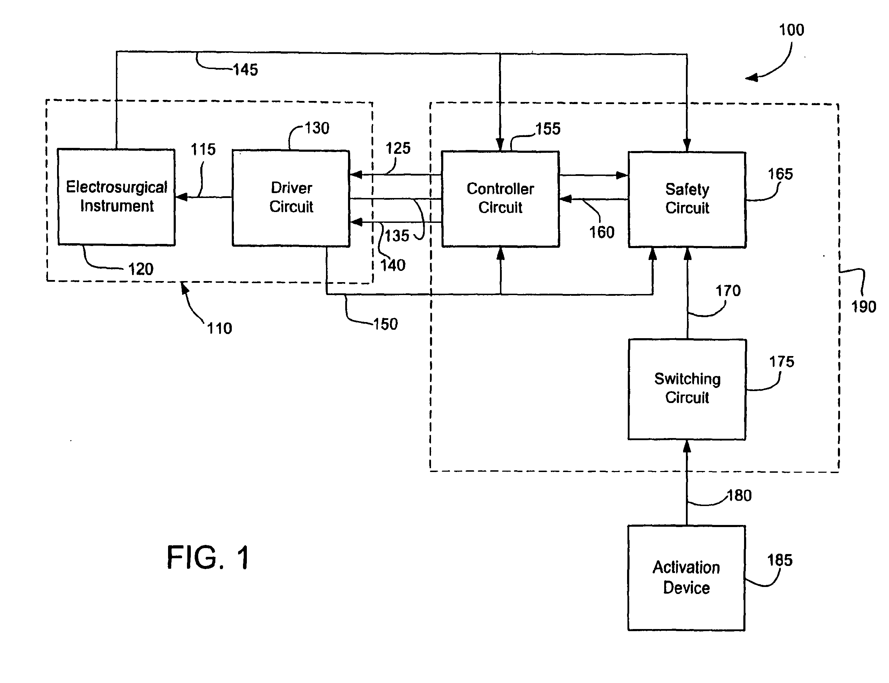

FIG. 1 is block diagram of a system for activating an electrosurgical instrument in accordance with an embodiment of the present disclosure.

### DETAILED DESCRIPTION OF THE PREFERRED EMBODIMENTS

Preferred embodiments of the presently disclosed system for activating an electrosurgical instrument will now be described in detail with reference to the drawing, in which like reference numerals and characters designate identical or corresponding elements in the accompanying figure.

According to an embodiment of the present disclosure, an electrosurgical control system 100 is illustrated in FIG. 1. Electrosurgical control system 100 includes an electrosurgical assembly 110, a control assembly 190, and an activation device 185. Electrosurgical assembly 110 includes an electrosurgical instrument 120 and a related driver circuit 130 that are operatively coupled to an actuation mechanism or the control assembly 190 by an electrical or electro-mechanical connection 135. Typically, connection 135 is a cable including a plurality of connecting elements, such as wires, such that connection 135 is capable of communicating a number of different signals between electrosurgical assembly 110 and control assembly 190. Alternatively, connection 135 may be a wireless connection between electrosurgical assembly 110 and control assembly 190 that employs radio frequency or infrared communications.

In an exemplary embodiment, electrosurgical assembly 110 includes electrosurgical instrument 120 for performing a surgical procedure and driver circuit 130. Electrosurgical instrument 120 is operatively coupled to driver circuit 130 where driver circuit 130 communicates a supply signal 115 for operating electrosurgical instrument 120. Examples of electrosurgical instruments 120 include, but are not limited to, electrosurgical suction coagulators, electrosurgical pencils, electrosurgical forceps, electrosurgical vessel sealing instruments, surgical smoke removal instruments, and electrosurgical laparoscopic handsets. In turn, driver circuit 130 is operatively coupled to control assembly 190 by connection 135.

Preferably, control assembly 190 includes a controller circuit 155, a safety circuit 165, and a switching circuit 175. Control assembly 190 interfaces between activation device 185 and electrosurgical assembly 110. Specifically, controller circuit 155 is operatively coupled to driver circuit 130 by connection 135 for transmitting a power signal 125 and a control signal 140 to driver circuit 130. Internal to control circuit 155 is a power forming circuit that generates power signal 125. Advantageously, power signal 125 is capable of carrying the positive component for use with monopolar electrosurgical instruments, and is further capable of including the positive and return components of the power feed for use with bipolar electrosurgical instruments. Operation of driver circuit 130 is regulated by controller circuit 155 by a control signal 140. The starting, stopping, and regulation of driver circuit 130, and the resulting supply signal 115, is controllable by controller circuit 155 via control signal 140.

Further included in electrosurgical control system 100 are an instrument signal 145 and a monitoring signal 150. Instrument signal 145 originates in electrosurgical instrument 120 and is communicated to controller circuit 155 and safety circuit 165 for transmitting data representing selected parameters in and/or around electrosurgical instrument 120 (i.e. the supervisory monitoring information). The data that is transmitted from electrosurgical instrument 120 may include: internal and external temperature information; voltage information; current flow information; pressure and/or vacuum information; and fluid flow information.

Controller circuit 155 is programmable to vary control signal 140 and power signal 125 in response to data received on instrument signal 145. By communicating information representative of selected parameters in and/or around electrosurgical instrument 120 in real-time, controller circuit 155 is capable of adjusting operating parameters of surgical assembly 110 as a real-time response to changes in the operational conditions of electrosurgical instrument 120. As an example, if the temperature of body tissue in the vicinity of electrosurgical instrument 120 rises and reaches a selected value, this temperature data is transmitted in instrument signal 145 and relayed to control circuit 155 in real-time. In response, controller circuit 155 is programmable for alerting the operator to the changing condition using audible and/or visual warnings. Preferably, controller circuit 155 or safety circuit 165 is further capable of automatically reducing power signal 125 in response to changing temperature conditions, thereby reducing the heat generated by electrosurgical instrument 120 and providing an additional measure of equipment and personnel safety. In a preferred embodiment, controller circuitry 155 is able to alert operating personnel using audible and/or visual warnings that selected parameters in and/or around electrosurgical instrument 120 are not within a desired range. Safety circuit 165 is programmable to generate automatic responses to selected parameters of electrosurgical instrument 120 that are outside the desired range.

Information relating to operational parameters of driver circuit 130 is supplied in monitoring signal 150 that is communicated to controller circuit 155 and safety circuit 165. Operational parameters may include data corresponding to temperature values, voltage values, current flow values, pressure and/or vacuum values, and fluid flow data in driver circuit 130. Selected parameters (i.e. the supervisory monitoring information) are monitored by controller circuit 155 and safety circuit 165. By monitoring operational parameters in driver circuit 130 and comparing them to the monitored operational parameters of electrosurgical instrument 120, increased system reliability is achievable. Redundant monitoring of the same or related signals enables electrosurgical control system 100 to respond to discrepancies between parameters in electrosurgical instrument 120 and driver circuit 130 that indicate potential problems in electrosurgical assembly 110.

By way of example only, a rise in the measured current flow through driver circuit 130 without a corresponding rise in current flow through electrosurgical instrument 120 indicates that a problem exists in electrosurgical assembly 110. In response to such a discrepancy, electrosurgical control system 100 is capable of generating audible and/or visual warnings to operating personnel. In other situations where the discrepancy indicates that a potentially hazardous condition exists, electrosurgical control system 100 is programmable to automatically reduce power and/or shut off electrosurgical assembly 110 to protect personnel and equipment.

A control signal 160 is generated by safety circuit 165 and is operatively coupled to an input of controller circuit 155. In the automatic modes of operation discussed hereinabove, safety circuit 165 modifies control signal 160 to execute the proper response to changing operational conditions as indicated by the monitored parameters. Additionally, safety circuit 165 generates control signal 160 to initiate operation of electrosurgical control system 100 after particular criteria are satisfied. In a preferred embodiment of the present disclosure, safety circuit 165 is programmable for accomplishing the above-mentioned operations and additional ones hereinafter disclosed. In cooperation with controller circuit 155, switching circuit 175, and activation device 185, safety circuit 165 transmits safety control signal 160 to controller circuit 155 for operating electrosurgical assembly 110.

Preferably, safety circuit 165 includes a logic circuit that is programmable for accomplishing the above-listed functions. By using a programmable logic circuit, it is possible for safety circuit 165 to be adapted to a number of different electrosurgical instruments 120 and/or procedures. The logic circuit may preferably include one or more integrated circuits along with associated circuitry that accepts inputs from switching circuit 175, electrosurgical instrument 120, driver circuit 130, and/or stored data within safety circuit 165. However, it is possible to employ electro-mechanical, electrical, and/or electronic components to provide the desired functions. For example, the logic circuit will inhibit the generation of control signal 160 when there is no latch signal 170 present at the appropriate input to the logic circuit. The logic circuit may also be programmed to disregard latch signal 170 for a time period when activation device 185 is used for starting and stopping electrosurgical instrument 120. Other combinations and uses for the logic circuit within safety circuit 165 are envisioned in addition to the examples given above.

In addition, the logic circuit accepts the inputs from switching circuit 175, electrosurgical instrument 120, driver circuit 130, and the stored data within safety circuit 165, and processes the inputs to determine whether any unsafe and/or undesirable operating conditions exist. An unsafe and/or undesirable operating condition, such as overheating of driver circuit 130, will be processed by the logic circuit, thereby inhibiting the generation of control signal 160. The logic circuit may also receive supervisory monitoring inputs from electrosurgical instrument 120, controller circuit 155, switching circuit 175, and/or driver circuit 130 where these supervisory monitoring inputs reflect a real-time status of the various circuits. If a problem is communicated on one of the supervisory monitoring inputs, safety circuit 165 is programmable to prevent operation of electrosurgical instrument 120 and/or alert operating personnel that an unsafe condition exists. Warnings to operating personnel may include audible and/or visual indications as to the unsafe condition. Safety circuit 165 may also be programmed to provide audible and/or visual indications to the operator when switching circuit 175 communicates a continuous latch signal 170 to safety circuit 165 indicating that a latch circuit in switching circuit 175 is in a "continuous" operating mode. Such information is communicated to the operator, via audible or visual indications, which allows the operator to release activation device 185, thereby reducing the operator's stress and/or fatigue while electrosurgical instrument 120 continues to operate.

Operation of electrosurgical assembly 110 including electrosurgical instrument 120 is initiated by the operating personnel actuating activation device 185. Examples of activation device 185 include, but are not limited to, hand switches, foot switches, and pushbuttons. Once actuated by the operating personnel, activation device 185 generates trigger signal 180 that is communicated to switching circuit 175. In response to the inputted trigger signal 180, switching circuit 175 generates and communicates a latch signal 170 to safety circuit 165 indicating a request to activate the electrosurgical instrument 120. Switching circuit 175 is programmable for a number of different operating configurations.

As can be appreciated, a user can selectively utilize the electrosurgical instrument 120 in an "on/off" mode by continuously depressing and releasing activation device 185 (e.g. switch) during surgery. Alternatively, the user may initiate a continually activated mode of operation, if desired, by either: 1) manually switching to a continual "ON" mode when the activation device 185 is depressed and an "OFF" mode when pressed again; or 2) enabling the electrosurgical instrument 120 to automatically switch to a "continuous" mode after a prescribed condition or time is satisfied, thus allowing the user to release the activation device 185 and reduce operating fatigue. As mentioned above, a tone or visual signal may be employed to alert the user of the changes in operating mode (i.e. on/off to continuous). It is envisioned that the continuous mode may be deactivated at any time by either: 1) disarming the continuous mode (i.e. not selecting continuous mode as a possible operating condition) at the onset of the surgery; or 2) if during surgery in a continuous mode, manually deactivating the continuous mode by depressing the activation device 185 (e.g. switch).

When electrosurgical control system 100 is being used in the continuous operating mode, activation device 185 initially generates a trigger signal 180 that is communicated to an input of switching circuit 175. In response, switching circuit 175 generates a latch signal 170 that is communicated to safety circuit 165 to initiate a continuous mode of operation. Inside safety circuit 165, the logic circuit is conditioned to accept latch signal 170 as an indication to initiate a continuous mode of operation, thereby when latch signal 170 is applied to an input of safety circuit 165, the logic circuit will process latch signal 170 and generate control signal 160. In turn, control signal 160 is communicated to controller circuit 155 to produce power signal 125 and turn electrosurgical instrument 120 on. In addition, safety circuit 165 may provide audible and/or visual indications to the operator that electrosurgical instrument 120 has been turned on. After electrosurgical instrument 120 is operating in the continuous mode of operation, the operator may release activation device 185, thereby interrupting trigger signal 180 and latch signal 170. Once latch signal 170 is no longer applied to an input of safety circuit 165, the logic circuit is prepared to accept the subsequent latch signal 170 as an indication to interrupt the continuous mode of operation as hereinabove discussed. Consequently, activation device 185 may be deactivated, thereby ceasing the generation of trigger signal 180 while electrosurgical instrument 120 continues to operate.

A subsequent actuation of activation device 185 while electrosurgical control system 100 is in the continuous operating mode generates another trigger signal 180 that is in turn coupled to an input of switching circuit 175 for generating latch signal 170. Latch signal 170 is then communicated to safety circuit 165. Preferably, latch signal 170 is inputted to the logic circuit of safety circuit 165. The logic circuit processes the inputted latch signal 170 along with other selected parameters. In this situation, the logic circuit will accept and process the inputted latch signal 170 as an indication to shut down electrosurgical instrument 120. The logic circuit will inhibit generation of control signal 160. In addition, the logic circuit will now be prepared to respond to another inputted latch signal 170 as an indication to reinitiate a continuous mode of operation. Lacking an input control signal 160, controller circuit 155 will terminate generation of power signal 125, thereby turning off electrosurgical instrument 120. Further still, safety circuit 165 may provide audible and/or visual indications to the operator that electrosurgical instrument 120 has been turned off.

After ceasing the continuous mode of operation of electrosurgical instrument 120, the operator can reinitiate the continuous mode of operation by actuating activation device 185 and generating trigger signal 180. Trigger signal 180 is communicated to switching circuit 175 for generating latch signal 170 in response to the inputted trigger signal 180. Inside safety circuit 165, the logic circuit is conditioned to accept latch signal 170 as an indication to reinitiate a continuous mode of operation, thereby when latch signal 170 is applied to an input of safety circuit 165, the logic circuit will process latch signal 170 and generate control signal 160. In turn, control signal 160 is communicated to controller circuit 155 to produce power signal 125 and turn electrosurgical instrument 120 on. In addition, safety circuit 165 may provide audible and/or visual indications to the operator that electrosurgical instrument 120 has been turned on. After electrosurgical instrument 120 is operating in the continuous mode of operation, the operator may release activation device 185, thereby interrupting trigger signal 180 and latch signal 170. Once latch signal 170 is no longer applied to an input of safety circuit 165, the logic circuit is prepared to accept the subsequent latch signal 170 as an indication to interrupt the continuous mode of operation as hereinabove discussed.

In one embodiment, latch circuit 175 generates latch signal 170 upon actuation of activation device 185 for a predetermined period of time. After a selected period of time, that may be selectively programmable for the specific procedure being performed, latch circuitry within switching circuit 175 latches-in for continuously generating latch signal 170, which permits continuous operation of electrosurgical instrument 120. If trigger signal 180 is interrupted, for example if there is no further actuation of activation device 185, electrosurgical instrument 120 will continue to operate provided that latch signal 170 is being communicated to safety circuit 165 indicating that the sealing circuitry has been activated.

In addition, electrosurgical control system 100 is configurable for activation device 185 to be temporarily disconnected by programming the latch circuitry in switching circuit 175 to lockout trigger signal 180 for a specified period of time. This is especially useful when activation device 185 is used to start and stop operation of electrosurgical instrument 120. By using switching circuit 175 to provide lockout features similar to those found in safety circuit 165, it is possible to provide a layer of redundancy for locking out activation device 185 during pre-programmed periods of time. The latch circuitry may include electro-mechanical, electrical, and/or electronic components configured and adapted to provide the latching and/or timing features of switching circuit 175. Preferably, one or more integrated circuits along with associated circuitry will be employed that provide the desired latching and/or timing features of switching circuit 175.

In an alternative embodiment of electrosurgical control system 100, control assembly 190 may be combined into a single control circuit that replaces switching circuit 175, safety circuit 165, and controller circuit 155 and their associated functions. Preferably, the replacement control circuit will be an integrated circuit with associated circuitry that is programmable for a number of different operating conditions. It is further envisioned that control assembly 190, according to this embodiment, may be included in an electrosurgical generator for controlling electrosurgical instrument 120. Activation device 185 may be disposed on or in close proximity to the electrosurgical generator, depending on the type of electrosurgical instrument 120 that is being used (e.g. surgical smoke removal instruments). Alternately, activation device 185 may be disposed on or in proximity to electrosurgical instrument 120 (e.g. electrosurgical pencils). Furthermore, activation device 185 may be wirelessly coupled to the control assembly 190 via radio frequency or infrared communications.

Although the illustrative embodiments of the present disclosure have been described herein with reference to the accompanying drawing figure, it is to be understood that the disclosure is not limited to those precise embodiments, and that various other changes and modifications may be affected therein by one skilled in the art without departing from the scope or spirit of the disclosure. All such changes and modifications are intended to be included within the scope of the disclosure.

The following numbered paragraphs provide further conceptual disclosure of the present subject matter.
1. An electrosurgical control system comprising:
   an electrosurgical assembly for performing a surgical procedure;
   a control assembly operatively coupled to the electrosurgical assembly for controlling an operation of the electrosurgical assembly; and
   an activation device operatively coupled to the control assembly for generating a trigger signal to the control assembly,
   wherein upon the control assembly receiving a single trigger signal, the control assembly activates the electrosurgical assembly in a continuous operation mode.
2. An electrosurgical control system according to paragraph 1, wherein the electrosurgical assembly comprises an electrosurgical instrument operatively coupled to a driver circuit where the driver circuit provides a supply signal for operating the electrosurgical instrument.
3. An electrosurgical control system according to paragraph 2, wherein the control assembly comprises a controller circuit operatively coupled to the driver circuit for transmitting a power signal and a control signal to the driver circuit.
4. An electrosurgical control system according to paragraph 3, wherein the power signal is capable of carrying a positive component for use with a monopolar electrosurgical instrument.
5. An electrosurgical control system according to paragraph 3, wherein the power signal is capable of carrying a positive and return component for use with a bipolar electrosurgical instrument.
6. An electrosurgical control system according to paragraph 3, wherein the control assembly further comprises a safety circuit operatively coupled to the controller circuit for transmitting a control signal to the controller circuit for controlling the electrosurgical assembly when predetermined operational conditions are met.
7. An electrosurgical control system according to paragraph 6, wherein the control assembly further comprises a switching circuit coupled to the safety circuit for generating a latch signal upon activation of the activation device.
8. An electrosurgical control system according to paragraph 7, wherein upon activation of the activation device for a predetermined time period, the switching circuit generates a continuous latch signal to operate the electrosurgical assembly in the continuous operating mode.
9. An electrosurgical control system according to paragraph 8, wherein upon a subsequent activation of the activation device, the switching circuit stops generating the continuous latch signal, wherein the electrosurgical assembly is deactivated.
10. An electrosurgical control system according to paragraph 1, wherein the control assembly is coupled to the electrosurgical assembly wirelessly.
11. An electrosurgical control system according to paragraph 1, wherein the activation device is coupled to the control assembly wirelessly.
12. An electrosurgical generator comprising:
   a control assembly for controlling an operation of an electrosurgical assembly, the electrosurgical assembly performing a surgical procedure,
   wherein upon the control assembly receiving a single trigger signal, the control assembly activates the electrosurgical assembly in a continuous operation mode.
13. An electrosurgical generator according to paragraph 12, further comprising an activation device for generating the trigger signal.
14. An electrosurgical generator according to paragraph 13, wherein the activation device is disposed externally from the generator.
15. An electrosurgical generator according to paragraph 14, wherein the activation device communications to the control assembly wirelessly.
16. An electrosurgical generator according to paragraph 13, wherein the electrosurgical assembly comprises an electrosurgical instrument operatively coupled to a driver circuit where the driver circuit provides a supply signal for operating the electrosurgical instrument.
17. An electrosurgical generator according to Paragraph 16, wherein the control assembly comprises a controller circuit operatively coupled to the driver circuit for transmitting a power signal and a control signal to the driver circuit.
18. An electrosurgical generator according to paragraph 17, wherein the control assembly further comprises a safety circuit operatively coupled to the controller circuit for transmitting a control signal to the controller circuit for controlling the electrosurgical assembly when predetermined operational conditions are met.
19. An electrosurgical generator according to paragraph 18, wherein the control assembly further comprises a switching circuit coupled to the safety circuit for generating a latch signal upon activation of the activation device.
20. An electrosurgical generator according to paragraph 19, wherein upon activation of the activation device for a predetermined time period, the switching circuit generates a continuous latch signal to operate the electrosurgical assembly in the continuous operating mode.
21. An electrosurgical generator according to paragraph 20, wherein upon a subsequent activation of the activation device, the switching circuit stops generating the continuous latch signal, wherein the electrosurgical assembly is deactivated.
22. A method for controlling an electrosurgical instrument in a continuous operating mode, the method comprising the steps of:
   activating an activation device for a predetermined period of time;
   generating a latch signal after the predetermined period of time;
   generating a control signal upon receiving the latch signal to activate the electrosurgical instrument; and
   deactivating the activation device, wherein the electrosurgical instrument will operate in a continuous operating mode after the deactivation.
23. A method according to paragraph 24, further comprising the steps of:
   reactivating the activation device;
   generating a subsequent latch signal; and
   stopping the generation of the control signal to stop operation of the electrosurgical instrument.

## Claims

1. A method for controlling an electrosurgical instrument, the method comprising the steps of:
activating an activation device for a predetermined period of time;
generating a latch signal after the predetermined period of time;
receiving the latch signal to initiate activation of the electrosurgical instrument; and
deactivating the activation device in a manner such that the electrosurgical instrument operates in a continuous operating mode after the deactivation.

2. A method according to claim 1, further comprising the steps of:
reactivating the activation device; and
generating a subsequent latch signal to deactivate the electrosurgical instrument.

3. A method according to claim 1 or 2, wherein the method is for controlling an electrosurgical instrument in a continuous operating mode.

4. A method according to claim 1, 2 or 3, wherein receiving the latch signal to initiate activation of the electrosurgical instrument comprises generating a control signal upon receiving the latch signal to activate the electrosurgical instrument.

5. A method according to any one of claims 1-4, wherein deactivating the activation device comprises deactivating the activation device, wherein the electrosurgical instrument will operate in a continuous operating mode after the deactivation.

6. A method according to any one of claims 2-5, wherein generating a subsequent latch signal to deactivate the electrosurgical instrument comprises generating a subsequent latch signal; and stopping the generation of the control signal to stop generation of the electrosurgical instrument.

7. An electrosurgical control system comprising:
an electrosurgical instrument;
an activation device which upon activation generates a trigger signal and upon deactivation interrupts the trigger signal;
a switching circuit which upon communication of the trigger signal to the switching circuit generates a latch signal; and
a safety circuit comprising a logic circuit which is conditioned to initiate upon communication of a first latch signal to the safety circuit a continuous mode of operation of the electrosurgical instrument.

8. An electrosurgical control system according to claim 7,
wherein the logic circuit is further conditioned to interrupt the continuous mode of operation of the electrosurgical instrument upon communication to the safety circuit of a subsequent latch signal.
